# EUROPEAN PATENT APPLICATION

(11) **EP 3 753 563 A1**
(43) Date of publication of application: **23.12.2020**
(21) Application number: 20180738.5
(22) Date of filing: 18.06.2020
(51) Int. Cl.: A61K 31/522, A61P 31/12, A61P 31/14, A61P 31/16, A61P 31/20, A61P 31/22, A61P 35/00

(54) **ANTI-HERPESVIRUS GUANOSINE ANALOGUE FOR THE INHIBITION OF BASAL AUTOPHAGY**

(30) Priority: 20.06.2019 EP 19181522
(71) Applicant: Helmholtz-Zentrum für Infektionsforschung GmbH, 38124 Braunschweig (DE)
(72) Inventor: Fichtner, Janine, 38550 Isenbüttel (DE); Schmitz, Ingo, 38104 Braunschweig (DE); Wirth, Manfred, 38300 Wolfenbüttel (DE)
(74) Representative: Hertin und Partner Rechts- und Patentanwälte PartG mbB

(57) **Abstract**

The invention relates to an anti-herpesvirus guanosine analogue for use as a medicament to inhibit basal autophagy in the treatment and/or prevention of a medical condition in which basal autophagy is involved in the pathogenesis of said condition. In preferred embodiments, said analogue inhibits basal autophagy but does not inhibit induced autophagy or inhibits induced autophagy at negligible levels. The medical condition can be an infectious disease, in which basal autophagy in the host cell of a disease-causing infectious agent supports infection and/or propagation of the infection, which is inhibited by said use of the analogue. Alternatively, the medical condition is a cancer, wherein the cancer exhibits basal autophagy in support of cancer initiation, cancer cell survival, proliferation, drug resistance and/or motility, which is inhibited by said use of the analogue. Also, the medical condition can be a neurodegenerative disease, in which basal autophagy supports pathogenesis of said condition, preferably wherein the condition is Neurodegenerative Vacuolar Storage Disease (NVSD).

## Description

The invention relates to the field of treatment of medical conditions in which basal autophagy is involved using guanosine analogues.

The invention therefore relates to an anti-herpesvirus guanosine analogue for use as a medicament to inhibit basal autophagy in the treatment and/or prevention of a medical condition in which basal autophagy is involved in the pathogenesis of said condition. In preferred embodiments, said analogue inhibits basal autophagy but does not inhibit induced autophagy or inhibits induced autophagy at negligible levels. The medical condition can be an infectious disease, in which basal autophagy in the host cell of a disease-causing infectious agent supports infection and/or propagation of the infection, which is inhibited by said use of the analogue. Alternatively, the medical condition is a cancer, wherein the cancer exhibits basal autophagy in support of cancer initiation, cancer cell survival, proliferation, drug resistance and/or motility, which is inhibited by said use of the analogue. Also, the medical condition can be a neurodegenerative disease, in which basal autophagy supports pathogenesis of said condition, preferably wherein the condition is Neurodegenerative Vacuolar Storage Disease (NVSD).

### BACKGROUND OF THE INVENTION

Basal (macro)autophagy is an important degradation mechanism in animal cells. Basal autophagy is responsible for cellular homeostasis and quality control. Basal autophagy is also involved in disease mechanisms such as viral and bacterial infections, the development of certain neurodegenerative diseases and the supply of cancer cells. Due to its importance for the cell and the diseases mentioned above, autophagy has become an increasingly important research target for drug development in the last 10-15 years (Rubinsztein et al, Nature Rev Drug Discovery, 2012).

The detailed molecular and cell biological investigation of autophagy was carried out intensively in the 1990s in yeasts and in the 2000s in the mammalian system. In the meantime, more than 30 ATG (autophagy-related) proteins have been characterized.

In (macro)autophagy, a newly formed double membrane (phagophore) encloses unwanted material, which is then degraded and recycled after fusion of the autophagosome thus formed with the lysosome (= autophagolysosome): There is a basal (constitutive) and an induced form of autophagy. The basal autophagy of the animal cell is responsible for the removal of protein aggregates and defective or superfluous organelles. The more pronounced, induced form of autophagy is triggered by stress factors (e.g. supply bottlenecks such as amino acid deficiency and energy deficiency, as well as ER stress, reactive oxygen species (ROS) or infection by certain microorganisms) and ensures the survival of the cell by eliminating these states and bottlenecks.

Autophagy plays a special role in the infection of animal cells by microorganisms, e.g. viruses (Chiramel and Bartenschlager, Cells 2013). On the one hand, autophagy can be elicited upon infection with certain pathogens as a cellular defense mechanism in order to destroy the invaders (xenophagy, LAP). Usually, pathogens try to circumvent such destructive processes. On the other hand, certain microorganisms coopt the early steps in autophagy for their own purposes (e.g. remaining inside the vesicles as a shield, for vesicular transport, or as a lipid-rich replication site) and escape from degradation late in autophagy subsequently by inhibition of autophagic maturation. Support by the autophagic vesicles in egress of viruses has also been reported (EBV, lytic cycle).

It is known that certain viruses induce autophagy in order to benefit from the process. On the other hand, they have also developed mechanisms to counteract autophagy and thus avoid degradation in autolysosomes. Herpes viruses are an example of this. HSV-1 (herpes simplex virus 1, responsible for lip herpes) and VZV (varicella zoster virus) induce autophagy and this would stop virus replication (Yakoub and Shukla, Nature Sci Rep, 2015a). However, the viral protein ICP34.5 binds the central autophagy factor Beclin-1, which leads to the inhibition of autophagy. Thus, these virus types escape their destruction in autolysosomes. However, it has recently been reported that HSV-2 (responsible for genital herpes) requires the basal form of autophagy for productive replication, and both pharmacological and genetic inhibition of basal autophagy drastically interfere with virus formation (Yakoub and Shukla, Nature Sci Rep, 2015b).

Influenza A viruses are another example. It has been shown that some strains mostly exploit or switch on autophagy in the early stages of viral infection and benefit from it, preventing the maturation of autophagosomes for various reasons in the late stage of propagation.

Furthermore, virus families are known that specifically interact with the membranes formed during autophagy and use them as a site for their replication. Examples are hepatitis C virus (HCV) and the family of corona viruses.

Another field of interest involving basal autophagy are stem cells. Stem cells are dependent on basal autophagy for maintaining their stemness. Interestingly, cancer stem cells (CSCs) have higher rates of basal autophagy than (non-stem) cancer cells. CSCs locate close to solid tumors and are a target for anti-cancer drug discovery, since they have the potential for malignancy initiation, of tumor metastasis and enhanced chemotherapy resistance.

Cancer stem cells represent about 0.04 % of cells in tumors. CSCs have the potential for self-renewal but also the potential for initiation of malignancy, of tumor metastasis and enhanced chemotherapy resistance. Cancer stem cells share a number of biological hallmarks that are different from their normal-tissue counterparts and these might be taken advantage of for therapeutic benefits. For example, a temporal, dose-controlled interference with CSC biology via reduction of increased basal autophagy should have pronounced effects on CSC stemness and survival, while other cells might not be affected. The field of cancer stem cell biology is rapidly evolving. Markers for CSCs are defined (e.g. ALDH+) and CSC expansion and propagation is reported (Serra et al., Stem Cells Int, 2019).

Furthermore, neurodegenerative disorders represent another group of diseases in which basal autophagy is involved in pathogenesis. In particular, autophagy has been reported to play a critical role in Neurodegenerative Vacuolar Storage Disease (NVSD). In NVSD, basal autophagy is altered in Lagotto Romagnolo dogs with an ATG4D mutation leading to increased formation of autophagosomes. This is accompanied by large vacuole formation in brain cells and subsequent onset of NVSD (Syrviä et al. Vet Path 2017; Kyöstila et al. PLOS Gen, 2015). NVSD is a neurological disorder and besides Lysosomal Lipid Storage Disorders (LSD) the most prominent neurological disorder in this dog breed. In affected dogs NVSD manifests in progressive cerebellar ataxia, sometimes accompanied by episodic nystagmus and behavioral changes.

There are numerous substances that can modulate autophagy and they are often used in experimental approaches. However, there are only few clinically applied or marketed autophagy modulators. For example, chloroquine has long been used in combination with other substances to inhibit autophagy. In the past, chloroquine was used as an antimalarial agent. It inhibits the haem utilization by the pathogen. However, it also stops the enzymatic degradation of substances in the lysosomes by changing the pH value. This effect is unspecific and affects autophagy as well as other cellular processes.

The lack of specificity of autophagy modulators is a general problem. Almost all autophagy modulators have this disadvantage, since other cellular signaling pathways are almost always influenced, which means that the side effects of drug use are numerous. Exceptions are BeclinD11 (e.g. Tat-Beclin 1 D11 autophagy inducing peptide; a peptide comprising 11 amino acids, in the retero-inverso D-configuration, derived from Beclin 1 linked to the HIV Tat protein with a diglycine linker) and ATG16 protein (involved in the initiation phase of autophagosome formation), which specifically target interactions of important autophagy proteins.

Accordingly, there is a need in the art for an inhibitor or a drug that reduces or inhibits basal autophagy, but preferably does not influence the initiation or maturation of the autophagosomes in the case of stress-induced autophagy.

### SUMMARY OF THE INVENTION

In light of the prior art the technical problem underlying the present invention is to provide an inhibitor of basal autophagy. Another objective of the invention is to provide means for treating medical conditions in which autophagy plays a role in the pathogenesis of the disease. In some embodiments, the invention seeks to provide a specific inhibitor of basal autophagy that does not or not substantially interfere with induced autophagy, and which is safe to use in patients as a medicament in the treatment and/or prevention of a medical condition in which basal autophagy is involved in the pathogenesis of said condition.

This problem is solved by the features of the independent claims. Preferred embodiments of the present invention are provided by the dependent claims.

The invention therefore relates to an anti-herpesvirus guanosine analogue for use as a medicament to inhibit basal autophagy in the treatment and/or prevention of a medical condition in which basal autophagy is involved in the pathogenesis of said condition.

The anti-herpesvirus guanosine analogues of the invention can be used for the treatment and/or prevention of medical conditions that employ or use basal autophagy as a mechanism to promote disease progression and pathogenesis. Accordingly, the medical conditions to be treated by the invention differ from conditions that are associated with a malfunction of basal autophagy. Herein, anti-herpesvirus guanosine analogues may also be referred to as the compounds of the invention.

Anti-herpesvirus guanosine analogues, such as acycloguanosine, also known as acyclovir or aciclovir, are well-known active ingredients that reduce the replication and thus the proliferation of certain herpes viruses. The commonly known mechanism of action of these substances is due to its specificity for viral thymidine kinase, so that only viral DNA replication is affected, and cellular DNA replication remains unaffected.

The present invention is based on the entirely surprising finding that anti-herpesvirus guanosine analogues and in particular aciclovir have a second mechanism of action and surprisingly also inhibit basal (macro)autophagy, an important degradation mechanism in animal cells. This mode of action has not yet been described in the art and opens up entirely surprising applications of anti-herpesvirus guanosine analogues.

A further unexpected feature of the present invention is that the effective concentration of the anti-herpesvirus guanosine analogues of the invention is much lower than the effective concentration described for the inhibition of viral DNA replication. In fact, the inhibitory effect on basal autophagy is much more pronounced at these lower concentrations and is significantly reduced at the concentrations used for the thymidine inhibitory effect.

It was very surprising that the concentrations used therapeutically in the art for inhibiting viral DNA replication do not show substantial effects on autophagy. In contrast, the effect typically becomes more pronounced and effective upon employing substantially lower concentrations, which are almost ineffective against viral DNA replication.

The use of anti-herpesvirus guanosine analogues such as aciclovir for inhibiting basal autophagy is advantageous for several reasons. It represents a s a 'repurposing', in which an already approved drug can be used for new indications. Anti-herpesvirus guanosine analogues such as aciclovir have been on the market for more than 30 years and numerous efficacious modified derivatives have been developed since then.

The side effects of aciclovir in the high dosage used for inhibition of viral DNA synthesis are well known and are typically low, and related mostly to long-term treatment with high dosages. However, the lower concentrations to be employed in the context of the present invention, which may be in some embodiments about 20-fold lower, can be expected to be unproblematic with respect to adverse side effects, which will be surely lower than previously described for known indications. Due to the low dosage of an approved and well characterized drug with already low side-effect potential in high dosages, the present invention is expected to be safe and well tolerated. Thus, the application improves the therapeutic index.

Importantly, in some embodiments, aciclovir acts only on basal autophagy, while stress-induced autophagy is not or not substantially affected. Thus, for example the intracellular proliferation or replication of microorganisms that benefit from basal autophagy is affected, while induced autophagy is not affected by the inhibition, i.e. the cellular response to stressors is not impaired and remains intact, for example in the context of infection defense.

In embodiments of the invention said anti-herpesvirus guanosine analogue is selected from the group consisting of aciclovir, penciclovir, ganciclovir and/or analogues, derivatives, esters or prodrugs thereof, such as valaciclovir, valganciclovir and famciclovir.

It is a striking advantage of the present invention that known anti-herpesvirus guanosine analogues that have been safely employed in the treatment of patients can be used for the new medical use described herein. Importantly, for inhibiting basal autophagy only substantially reduced amounts of these compounds are necessary as compared to their known application. Therefore, the side-effects of compounds will also be substantially reduced or will not even occur in the context of the present invention, especially when short-term administration is applied.

The present invention is therefore defined, in some embodiments, by the identification of a novel mode of action of anti-herpesvirus guanosine analogues. This previously unknown and unsuggested mode of action enables novel applications of the class of active agents described herein. A novel clinical situation arises through this unexpected finding. In some embodiments, novel doses can be administered to patients, thereby substantially changing the way this class of agents has been previously administered. Furthermore, novel patient collectives may now be treated, for which no suitable therapy was previously known. Identifying a patient group based on basal autophagy defines a novel (sub)group of patients for whom suitable treatments may now be available.

In one embodiment of the invention, the anti-herpesvirus guanosine analogues are acycloguanosine analogues.

Preferably, the anti-herpesvirus guanosine analogue of the invention inhibits basal autophagy but does not inhibit induced autophagy or inhibits induced autophagy at negligible levels. In this context, inhibition at negligible levels refers to an inhibition such as a reduced level of induced autophagy that does not result in a relevant physiological or pathological effect.

It is an important feature of this embodiment that anti-herpesvirus guanosine analogues only inhibit basal autophagy, but not induced autophagy, since these two mechanisms have differential functions in disease pathology. Accordingly, the compounds of the invention enable an efficient and specific treatment of medical conditions associated with basal autophagy, while induced autophagy that may have a different function during pathogenesis remains unaffected. This is not possible by using known broad autophagy inhibitors, that cannot differentiate between different autophagy mechanisms. Accordingly, the present invention represents a significant advance in comparison to the state-of-the-art approaches using autophagy inhibitors for treating diseases associated with autophagy, meaning that autophagy is promoting disease progression and pathology.

In embodiments of the invention, the medical condition is an infectious disease, in which basal autophagy in the host cell of a disease-causing infectious agent supports infection and/or propagation of the infection, which is inhibited by said use of the analogue. The use of the compounds of the present invention for specifically inhibiting basal autophagy makes it possible to provide a targeted therapy of such infectious diseases, that are promoted by the cellular mechanism of basal autophagy.

As explained above, certain infectious diseases use basal autophagy as a mechanism to promote growth and replication of the infectious agent and there by promote disease progression. However, in other infectious diseases basal autophagy hinders disease progression. To add another level of complexity, in some cases basal autophagy can have different effects on pathogenesis of infectious diseases than induced autophagy, wherein this also depends on the specific infectious disease. Therefore, it is an important advancement to provide compounds that specifically inhibit basal macro autophagy so that these compounds can be used for treating such infectious diseases, that are promoted by basal autophagy, but not induced autophagy. This means that in infectious disease where basal autophagy is promoting disease, but induced autophagy is counteracting disease progression, basal autophagy can specifically and selectively be inhibited by means of the present invention, while the antimicrobial mechanism of induced autophagy can be maintained.

In embodiments of the invention, the infectious disease is selected from the group consisting of infections with Influenza A virus, Hepaptitis C virus (HCV), Staphylococcus aureus (S. aureus), Herpes Simplex Virus 2 (HSV-2), Epstein-Barr-Virus (EBV) and Coronaviruses (CoV), including severe acute respiratory syndrome-related coronavirus (SARS-CoV), SARS-CoV-2, Middle East respiratory syndrome-related coronavirus (MERS-CoV), human coronavirus OC43 (hCoV-OC43) and hCoV-229E. These particular infectious diseases have been shown to use basal autophagy to promote disease progression and replication of the infectious agent (HSV-2, EBV) or are suspected to depend on basal autophagy (Influenza A virus, Coronaviruses, HCV, Staphylococcus aureus) and exploit autophagy as such, and therefore represent preferred medical conditions to be treated by the anti-herpesvirus guanosine analogues of the present invention.

In further embodiments of the invention, the infectious disease is selected from the group consisting of infections with Hepaptitis C virus (HCV), Herpes Simplex Virus 2 (HSV-2), Epstein-Barr-Virus (EBV) and Coronaviruses (CoV), including severe acute respiratory syndrome-related coronavirus (SARS-CoV), Middle East respiratory syndrome-related coronavirus (MERS-CoV), human coronavirus OC43 (hCoV-OC43) and hCoV-229E.

In embodiments of the invention, the infectious disease is an infection with SARS-CoV-2. In embodiments, the infectious disease is COVID-19.

In embodiments of the invention, the infectious disease is an infection with Influenza A virus or Staphylococcus aureus. In the case of influenza A, it has been shown that these viruses can use autophagy during early stages of viral infection to promote viral replication and therefore represent a suitable target of the present invention. Similarly, it has been shown that Staphylococcus aureus uses basal autophagy for intracellular replication (Neumann et al. Autophagy. 2016 Nov;12(11):2069-2084).

In embodiments of the invention, the medical condition is cancer, and wherein
a. the cancer exhibits basal autophagy in support of cancer initiation, cancer cell survival, proliferation, drug resistance and/or motility, which is inhibited by said use of the analogue, and/or
b. the cancer has elevated levels of basal autophagy compared to a suitable control.

Certain cancers represent suitable medical condition that can be treated by the compounds of the present invention. It is known to the skilled person that certain cancer types exhibit enhanced basal autophagy activity as compared to other cancer cells or the healthy cells of origin of the cancer cells. As used herein, a cell of origin of a cancer refers to the physiologically occurring cell of the subject affected with the medical condition of the invention from which the cancer cells have initially differentiated through transformation.

Basal autophagy can be important for promoting survival, proliferation, drug resistance, motility, metastasis formation, and other required features of cancer cells that promote cancer disease progression and pathology. Accordingly, in such cancer cells exhibiting increased level of basal autophagy, inhibition of basal autophagy can lead to the therapeutic effect and slowing down of disease progression. Suitable cancers to be treated by the compounds of the present invention can be identified by comparing the level of basal autophagy of the cancer cells to suitable control cells, such as the healthy untransformed cell of origin of the respective cancer cells. The skilled person knows how to measure and compare the level of basal autophagy, for example by employing the reporter constructs used in the figures and examples disclosed herein or other established essays to determine the rate of basal autophagy in cells.

In a further embodiment of the invention,
- the cancer cells are resistant to one or more anticancer drug treatments, such as radiation therapy, chemotherapy or targeted immunotherapies; and/or
- the cancer cells exhibit elevated levels of autophagy as a cell survival-enhancing protective response to tumor microenvironmental stress, such as endoplasmic reticulum (ER) stress, hypoxia, oxidative stress and/or glucose deprivation;
- wherein said cancer is preferably selected from breast cancer, colorectal cancer, esophageal cancer, glioblastoma, hepatocellular carcinoma, leukemia and MCL, lung cancer, Pancreatic adenocarcinoma, prostate cancer, renal cell carcinoma or ovarian cancer.

The compounds of the present invention are particularly useful in treating cancer cells or cancers that have acquired resistance to other cancer therapies and therefore represents a significant advancement in cancer treatment. During the cause of cancer pathology, the transformed cells acquire further mutations and properties, that can make them resistant to ongoing treatments. For example, cancer cells can adapt to DNA damage induced by radiation therapy and/or can develop resistance mechanisms to chemotherapy and immunotherapies. However, cancer cells which are resistant to anticancer therapies often display increased levels of basal autophagy, which is required for the cells to survive and proliferate under these stress conditions. Accordingly, the compounds of the present invention can make such cells sensitive to treatments to which they had already acquired resistance.

Furthermore, cancer cells are able to adapt to stress conditions, such as a tumor microenvironment that can be hypoxic due to insufficient blood supply and fast growth of the tumor mass and oxidative stress. However, this adaptation often is supported and can depend on basal autophagy and therefore the treatment of the present invention is toxic to cancer cells that have employed this mechanism to adapt to the tumor microenvironment. Breast cancer, colorectal cancer, esophageal cancer, glioblastoma, hepatocellular carcinoma, leukemia and MCL, lung cancer, Pancreatic adenocarcinoma, prostate cancer, renal cell carcinoma or ovarian cancer have been observed to be frequently associated with increased levels of basal autophagy, which also often correlated with resistance to other anticancer treatments and drugs and adaptation to a toxic tumor microenvironment.

In further embodiments, the cancer cells are resistant to one or more anticancer treatments, such as radiation therapy, chemotherapy or targeted immunotherapies.

In embodiments, the cancer cells exhibit elevated levels of autophagy as a cell survival-enhancing protective response to tumor microenvironmental stress, such as endoplasmic reticulum (ER) stress, hypoxia, oxidative stress and/or glucose deprivation.

In another embodiment, the cancer is selected from breast cancer, colorectal cancer, esophageal cancer, glioblastoma, hepatocellular carcinoma, leukemia and MCL, lung cancer, Pancreatic adenocarcinoma, prostate cancer, renal cell carcinoma or ovarian cancer.

In another embodiment of the invention,
- the cancer cells are resistant to one or more anticancer treatments, such as radiation therapy, chemotherapy or targeted immunotherapies; and/or
- the cancer cells exhibit elevated levels of autophagy as a cell survival-enhancing protective response to tumor microenvironmental stress, such as endoplasmic reticulum (ER) stress, hypoxia, oxidative stress and/or glucose deprivation;
- wherein said cancer is preferably selected from breast cancer, colorectal cancer, esophageal cancer, glioblastoma, hepatocellular carcinoma, leukemia and MCL, lung cancer, Pancreatic adenocarcinoma, prostate cancer, renal cell carcinoma or ovarian cancer.

In embodiments, the cancer is a tumor or hematological cancer comprising cancer stem cells (CSCs), wherein the CSCs:
- exhibit basal autophagy in support of CSC pluripotency, CSC proliferation and/or CSC migration and/or invasion,
- exhibit elevated levels of basal autophagy compared to a suitable control, and/or
- are resistant to one or more anticancer drug treatments, such as radiation therapy, chemotherapy or targeted immunotherapies.

Cancer stem cells have been shown to depend on high rates of basal autophagy to maintain their stem cell potential. Due to their stem cell properties, CSCs represent a source of transformed cancer cells and can lead to initiation of the malignancies, even after an initial cancer outbreak could be treated effectively by certain anticancer therapies.

It is a great advantage of the present invention, that by specifically targeting the pathway of basal autophagy, which is required for maintenance of CSC stemness, CSCs can be effectively and specifically attacked by means of the present invention. This is particularly useful to prevent recurrence of a cancer that has been apparently effectively treated by other anticancer therapies, which do not specifically target CSCs.

Importantly, critical CSC properties such as pluripotency, proliferation, migration, and invasion have been shown to be associated with an increased level of basal autophagy in the cells. Accordingly, it is possible to reduce these highly pathogenic properties of CSCs by means of the present invention. CSCs exhibiting increased levels of basal autophagy can be identified by means known to the skilled person, for example by isolating the cells and measuring basal autophagy in comparison to a suitable control, such as the differentiated cancer cell or the untransformed healthy cell of origin of the CSC, by experimental tools known in the art. For example, the tandem reporter construct disclosed herein may be used in such a measurement. Since the compounds of the present invention can reduce or inhibit basal autophagy in CSCs, these become sensitive to other known anticancer therapies and therefore a combined treatment of the compounds of the present invention and for example radiation therapy, chemotherapy, and or targeted immunotherapy can be highly effective in eradicating CSC from a patient.

In further embodiments, the use of the compounds of the invention comprises combined administration of said analogue with one or more anticancer drug treatments, such as radiation therapy, chemotherapy or targeted immunotherapies. Combined administration of the compounds of the present invention with anticancer drug treatments, such as radiation therapy, chemotherapy and/or targeted immunotherapies is not only beneficial for targeting CSCs, but also for the treatment of cancers that are associated with an increased level of basal autophagy in general. As explained above, cancer cells can acquire additional properties including resistance to anticancer therapy during disease progression, which can be due to ongoing transformation and genetic instability, and which are often only possible in combination with an increased level of basal autophagy. Accordingly, combined administration of the compounds of the present invention and anticancer treatments can be highly effective, since the compounds of the invention inhibit basal autophagy, which is required in the cancer cells for maintaining pest electrical properties that enable anticancer treatment resistance. Therefore, the compounds of the present invention render cancer cells sensitive to other anticancer treatments.

In another embodiment, the condition is a neurodegenerative disease, in which basal autophagy supports pathogenesis of said condition, preferably wherein the condition is Neurodegenerative Vacuolar Storage Disease (NVSD).

NVSD is the neurodegenerative disorder found in dogs that is caused by mutations of the gene encoding for ATG4D. ATG4D is the cysteine protease that is involved in the basal autophagy pathway and plays an important role in regulating basal autophagy in the central nervous system and is required for neuronal homeostasis.

Mutations of ATG4D in dogs leads to increased formation of autophagosomes which is accompanied by large vacuole formation in neuronal cells of the central nervous system which is followed by onset of NVSD. NVSD clinically leads to uncoordinated movements due to neurological impairment.

In the context of the present invention, the compounds disclosed herein can be used for the treatment of NVSD in dogs and of related neurological disorders in other species associated with vacuole formation and increased autophagosome formation. In this context, the compounds of the invention can interfere with increased basal autophagy observed in these neurological diseases by repeated and/or time restricted administration. Administration may occur before or after diagnosis and before or after onset of the disease. In dogs, NVSD usually occurs by the appearance of clinical symptoms between the age of four months to four years.

Furthermore, in embodiments of the invention the subject is a mammal, preferably a human or canine.

In embodiments of the invention, the amount of analogue administered to a subject is:
a. below a therapeutically effective dose for treating Herpes Simplex Virus 1 (HSV-1) or varicella-zoster virus (VZV) infection in a subject; and/or
b. insufficient for inhibiting viral thymidine kinase in a subject infected with Herpes Simplex Virus 1 (HSV-1) or varicella-zoster virus (VZV).

In further embodiments, the amount of analogue administered to a subject is at least 5 times lower, preferably 10 times, more preferably 20 times lower, than (a) a therapeutically effective dose for treating HSV-1 or VZV infection in a subject, or (b) a dose effective in inhibiting viral thymidine kinase in a subject infected with HSV-1 or VZV.

The compounds of the invention have at least partially been used and approved for the treatment of herpes virus infection. However, it is a great advantage that in the context of the present invention the anti-herpes virus guanosine analogues can be used at much lower concentrations and administration can occur in much lower dosages as compared to the treatment of herpes viruses. Preferably, the dosage of the compounds of the invention is based on established treatments using aciclovir, which is probably the best studied anti-herpesvirus guanosine analogue.

It has been shown, that inhibition of basal autophagy by the compounds of the invention is most efficient at concentrations that are in the range of 5 to 100 fold lower than concentrations used for inhibiting purpose virus replication. In embodiments of the invention, the amount of the compounds of the invention used for treating a subject suffering from a medical condition as disclosed herein is about 2, 3, 4, 5, 6, 7, 8, 9, 10, 11, 12, 13, 14, 15, 16, 17, 18, 19, 20, 21, 22, 24, 23, 26, 25, 27, 28, 29, 30, 31, 32, 33, 34, 35, 36, 37, 38, 39, 40, 41, 42, 45, 46, 47, 48, 49, 50, 52,54,56,58,60,62,64,66,68,70,72,74,76,78,80,85,90,95,100,110,120,130,140,150, 160, 170, 180, 190, 200, 210, 220, 230, 240, 250, 260, 270, 280, 290, 300, 310, 320, 330, 340, 350, 360, 370, 380, 390, 400, 420, 440, 460, 480, 500, 520, 540, 560, 580, 600, 620, 640, 660, 680, 700, 720, 740, 760, 780, 800, 820, 840, 860, 880, 900, 920, 940, 960, 980, 1000 fold lower than the amount administered to a patient suffering from an herpes virus infection.

In particular, it is shown in the examples disclosed herein that inhibition of basal autophagy is most efficient at a concentration of 0.05 µM as compared to standard concentrations for the addition of herpes virus replication which are in the range of 5 to 20 µM. The EC 50 concentration of aciclovirfor HSV inhibition has been determined as 1.3 µM. Accordingly, the most effective amount of the compound of the invention was 400-fold reduced as compared to 20 µM, 100 fold reduced as compared to 5 µmol are, and 26 fold reduce as compared to 1.3 µM. Based on these experimental values, the skilled person can determine suitable dosages of the compounds to be used in the context of the present invention.

It is a great advantage of the invention, that due to the substantially reduced dosage of the compounds unwanted and toxic side effects such as neurological side effects and impairment of the kidney function that have been observed for the treatment of herpes virus infections can be prevented and are extremely unlikely to occur.

Accordingly, it is preferred that the compounds of the invention are administrated to a subject in one or more dosages that result in a concentration of the compound in the target tissue, such as for example a tumor mass or a tumor cell, or an infected tissue that is in the range of 0.01 - 0,5 µM. Preferably, the concentration in the target tissue is about 0.005, 0.006, 0.007, 0.008, 0.009, 0.01, 0.012, 0.014, 0.016, 0.018, 0.02, 0.022, 0.024, 0.026, 0.028, 0.03, 0.032, 0.034, 0.036, 0.038, 0.04, 0.042, 0.044, 0.046, 0.048, 0.05, 0.052, 0.054, 0.056, 0.058, 0.06, 0.062, 0.064, 0.066, 0.068, 0.069, 0.07, 0.072, 0.074, 0.076, 0.078, 0.08, 0.082, 0.084, 0.086, 0.088, 0.09, 0.092, 0.094, 0,096, 0.098, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1.0 µM.

Administration of the compounds of the invention may occur through various administration routes. Preferred routes of administration in the context of the invention are topical administration, inhalation, direct tissue injection, oral administration or intravenous administration. Further aspects concerning the route of administration and the effective amounts are defined below.

For the treatment of herpes virus, patients are treated with formulations of anti-herpes virus guanosine analogues and the concentration of about 5 mg/mL to 50 mg/mL, wherein the formulations can be liquid solutions for oral or intravenous administration or creams for topical administration or others.

Accordingly, the formulations used in the context of the invention may have reduced concentrations in the fold range as disclosed above for the total amounts of active compound administered. However, also formulations of similar concentration may be used and can be administered in reduced volumes.

Daily dosages of anti-herpes virus guanosine analogues for the treatment of herpes virus infection are usually in the range of 5 to 100 mg per kilogram body weight. However, the dosage depends on the route of administration, the age and the health status of the subject and further variables that can be taken into account by skilled person.

Preferably, in case of intravenous administration of a solution of anti-herpes virus guanosine analogues, the daily dose for adults suffering from HSV-1 or VZV infection is in the range of 15 to 30 mg per kilogram body weight, depending on the health status and comorbidities of the patient. In case of oral administration of tablets containing anti-herpes virus guanosine analogues, the daily dose can be as high as 4000 mg per day for an adult, usually about 2400 mg per day.

In some embodiments, the invention relates to an anti-herpesvirus guanosine analogue for use as a medicament as described herein, wherein:
- the dose of analogue, preferably aciclovir, administered is less than 5 mg/kg subject body weight per dose, for example about 3 mg/kg subject body weight or less per dose, preferably 1 mg/kg subject body weight or less per dose, more preferably 0.5 mg/kg subject body weight or less per dose; and/or
- wherein the analogue, preferably aciclovir, is administered in a liquid pharmaceutical composition at a concentration of less than 25 mg/ml, such as 10 mg/ml or less, preferably 2.5 mg/ml or less, more preferably 1 mg/ml or less, or is administered in a solid pharmaceutical composition comprising less than 200 mg, such as 100 mg or less, preferably 50 mg or less, more preferably 10 mg or less.

In some embodiments, the compounds of the invention are administered in a composition that enables specific targeting of pathogenic cells, i.e. infected cells in case of an infectious disease or cancer cells or CSCs in case of a cancer, in order to reduce unintended effects of the drug to non-diseased cells. For example, the compounds may be delivered using vesicles with membrane-incorporated cell-specific receptors/ligands or binding-molecules. The skilled person is able to select suitable delivery vehicles that enable targeted administration from the many known delivery tools known in the art. Non-limiting examples encompass the targeted administration of receptor/ligand or CPP cell targeting peptides to diseased cells using smart delivery of drugs e.g. via PLGA/PEG based nanoparticles or polyelectrolyte microcapsules.

### DETAILED DESCRIPTION OF THE INVENTION

All cited documents of the patent and non-patent literature are hereby incorporated by reference in their entirety.

The present invention is directed to an anti-herpesvirus guanosine analogue for use as a medicament to inhibit basal autophagy in the treatment and/or prevention of a medical condition in which basal autophagy is involved in the pathogenesis of said condition.

As used in the context of the invention, the term "anti-herpesvirus guanosine analogue" refers, in some embodiments, to a compound according to formula (I) 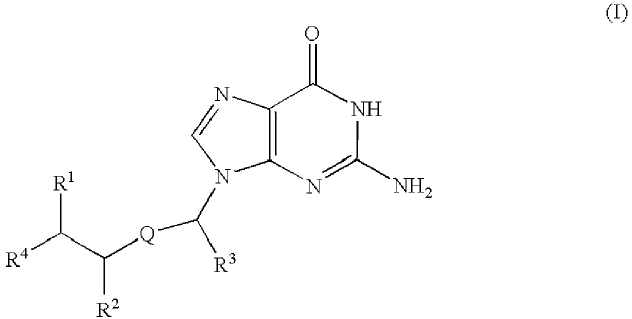 wherein R¹ is H, O, (C₁-C₄)alkyl, (C₃-C₆)cycloalkyl, hydroxy(C₁-C₄)alkyl, benzoyl(C₁-C₄)alkyl or phenyl; R² is H, OH, hydroxy(C₁-C₄)alkyl, (C₁-C₄)alkyl or -CH₂R^{a}; R³ is H, (C₁-C₄)alkyl or hydroxy(C₁-C₄)alkyl; R⁴ is OH or R^{a}; wherein each R^{a} is independently -Q-X-Y-Z₍ₙ₎₋R; each Q is independently S or O or C; each X, Y and Z is independently Met, Val, Thr, Tyr, Trp, Ser, Ala or Gly; each R is independently H or an amino-protecting group; and each n is independently 0 or 1; wherein at least one of R² is -CH₂R^{a} or R⁴ is R^{a}; or a pharmaceutically acceptable salt thereof. In this context, alkyl denotes both straight and branched groups; but reference to an individual radical such as "propyl" embraces only the straight chain radical, a branched chain isomer such as "isopropyl" being specifically referred to. The term "amino acid," comprises the residues of the natural amino acids (e.g. Ala, Arg, Asn, Asp, Cys, Glu, Gln, Gly, His, Hyl, Hyp, Ile, Leu, Lys, Met, Phe, Pro, Ser, Thr, Trp, Tyr, and Val). The terms "amino acid" and particular amino acids (e.g. Met, Val, Thr, Tyr, Trp, Ser, Ala, or Gly) include amino acid residues and include both the D and L stereoisomeric forms.

Furthermore, the anti-herpesvirus guanosine analogue of the invention can be selected from the group consisting of aciclovir, penciclovir, ganciclovir and/or analogues, derivatives, esters or prodrugs thereof, such as valaciclovir, valganciclovir and famciclovir.

Aciclovir is also called acycloguanosine and is the compound according to the following formula (II):

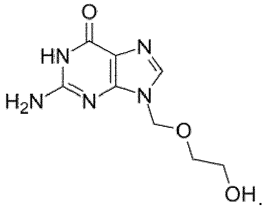

Aciclovir (ACV), also known as acyclovir, is an antiviral medication. It is primarily used for the treatment of herpes simplex virus infections, chickenpox, and shingles. Other uses include prevention of cytomegalovirus infections following transplant and severe complications of Epstein-Barr virus infection. It can be taken by mouth, applied as a cream, or injected. Common side effects include nausea and diarrhea. Potentially serious side effects include kidney problems and low platelets. Greater care is recommended in those with poor liver or kidney function. It is generally considered safe for use in pregnancy with no harm having been observed. It appears to be safe during breastfeeding. Aciclovir is a nucleoside analogue that is similar to guanosine. It works by decreasing the production of the virus's DNA.

Penciclovir is the compound according to the following formula (III):

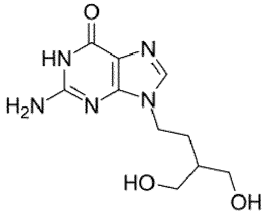

Penciclovir is a guanosine analogue antiviral drug used for the treatment of various herpesvirus infections. It is a nucleoside analogue which exhibits low toxicity and good selectivity. Because penciclovir is absorbed poorly when given orally (by mouth) it is more often used as a topical treatment. It is the active ingredient in the cold sore medications Denavir (NDC 0135-0315-52), Vectavir and Fenivir.

Famciclovir is a prodrug of penciclovir with improved oral bioavailability.

Ganciclovir is the compound according to the following formula (IV):

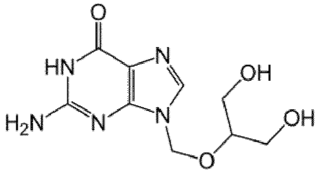

Ganciclovir is an antiviral medication used to treat cytomegalovirus (CMV) infections. A prodrug form with improved oral bioavailability (valganciclovir) has also been developed.

In some embodiments, the anti-herpesvirus guanosine analogue is 9-Carboxymethoxymethylguanine (CMMG), which is the principal metabolite of the antiviral drug acyclovir (and its prodrug valacyclovir).

As used herein, the term "analogue" refers to a structural analogue or analog, also known as a chemical analog or simply an analog. An analogue is a compound having a structure similar to that of another compound, but differing from it in respect to a certain component. It can differ in one or more atoms, functional groups, or substructures, which are replaced with other atoms, groups, or substructures. Structural analogs are often isoelectronic. In drug discovery either a large series of structural analogs of an initial lead compound are created and tested as part of a structure-activity relationship study or a database is screened for structural analogs of a lead compound.

In the context of the invention, the term analog in particular refers to functional analogs, which are chemical compounds that have similar physical, chemical, biochemical, and/or pharmacological properties. Functional analogs are not necessarily structural analogs with a similar chemical structure. An example of pharmacological functional analogs are morphine, heroin and fentanyl, which have the same mechanism of action, but fentanyl is structurally quite different from the other two.

A derivative is a compound that is derived from a similar compound by a chemical reaction. In some contexts, the term derivative is used to refer to a compound that can arise from another compound, if one atom or group of atoms is replaced with another atom or group of atoms, but modern chemical language more frequently uses the term "structural analog" for this meaning to avoid ambiguity. In biochemistry, "derivative" is used for compounds that can be formed from the precursor compound.

An "ester" is a chemical compound derived from an acid (organic or inorganic) in which at least one -OH (hydroxyl) group adjacent to C=O is replaced by an -O-alkyl (alkoxy) group. Usually, esters are derived from a carboxylic acid and an alcohol. Esters typically comprise the formula R(C=O)O-R', wherein R and R' are any given chemical moiety, preferably R' is an alkyl group. Alternatively, the chemical formula of an organic ester can take the form RCO2R', where R and R' are preferably the hydrocarbon parts of the carboxylic acid and the alcohol, respectively.

A prodrug is a medication or compound that, after administration, is metabolized (i.e., converted within the body) into a pharmacologically active drug. Inactive prodrugs are pharmacologically inactive medications that are metabolized into an active form within the body. Instead of administering a drug directly, a corresponding prodrug might be used instead to improve how a medicine is absorbed, distributed, metabolized, and excreted. Prodrugs are often designed to improve bioavailability when a drug itself is poorly absorbed from the gastrointestinal tract. A prodrug may be used to improve how selectively the drug interacts with cells or processes that are not its intended target. This reduces adverse or unintended effects of a drug, especially important in treatments like chemotherapy, which can have severe unintended and undesirable side effects. Prodrugs can be classified into two major types, based on how the body converts the prodrug into the final active drug form. Type I prodrugs are bioactivated inside the cells (intracellularly). Examples of these are anti-viral nucleoside analogs that must be phosphorylated and the lipid-lowering statins. Type II prodrugs are bioactivated outside cells (extracellularly), especially in digestive fluids or in the body's circulatory system, particularly in the blood. Examples of Type II prodrugs are salicin (described above) and certain antibody-, gene- or virus-directed enzyme prodrugs used in chemotherapy or immunotherapy. Both major types can be further categorized into subtypes, based on factors such as (Type I) whether the intracellular bioactivation location is also the site of therapeutic action, or (Type 2) whether or not bioactivation occurs in the gastrointestinal fluids or in the circulation system.

Valaciclovir is the compound according to the following formula (V):

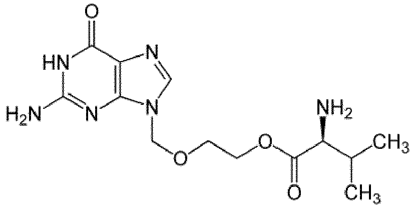

Valaciclovir, also spelled valacyclovir, is an antiviral medication used to treat outbreaks of herpes simplex or herpes zoster (shingles). It is also used to prevent cytomegalovirus following a kidney transplant in high risk cases. It is taken by mouth. Common side effects include headache and vomiting. Severe side effects may include kidney problems. Use in pregnancy appears to be safe. Valaciclovir is the L-valyl ester of aciclovir. It is a prodrug, which works after being converted to aciclovir in a person's body.

Valganciclovir can be a (*S,S*)-Diastereomer (Formula VI) or a (*S,R*)-Diastereomer (Formula VII) according to the following formulas:

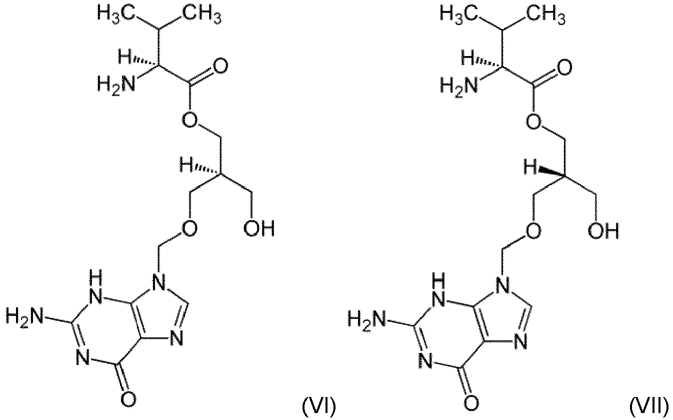

Valganciclovir is sold under the brand name Valcyte among others and is an antiviral medication used to treat cytomegalovirus (CMV) infection in those with HIV/AIDS or following organ transplant. It is often used long term as it only suppresses rather than cures the infection. Valganciclovir is taken by mouth. Common side effects include abdominal pain, headaches, trouble sleeping, nausea, fever, and low blood cell counts. Other side effects may include infertility and kidney problems. When used during pregnancy, it causes birth defects in some animals. Valganciclovir is the L-valyl ester of ganciclovir and works when broken down into ganciclovir by the intestine and liver.

Famciclovir is the compound according to the following formula (VIII):

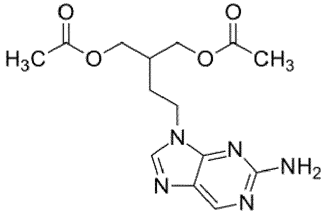

Famciclovir is a guanosine analogue antiviral drug used for the treatment of various herpesvirus infections, most commonly for herpes zoster (shingles). It is a prodrug form of penciclovir with improved oral bioavailability. Famciclovir is marketed under the trade name Famvir (Novartis).

The abbreviation "ACV" refers to acyclovir. The abbreviation "GCV" refers to ganciclovir. Amino acids are referred to by their standard three-letter abbreviations. Peptide sequences are written left to right from the amino to the carboxy terminus. Peptide esters of acyclovir wherein the hydroxyethoxymethyl group is esterified are referred to herein by abbreviations such as "Gly-Val-ACV."

This is a compound of formula (I) in which the carboxy group of the Valine residue of the Gly-Val dipeptide is esterified to the hydroxy group of acyclovir to form the R⁴ group of a compound of formula (I) as shown below.

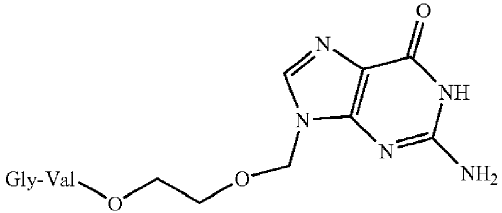

It will be appreciated by those skilled in the art that compounds of the invention having a chiral center may exist in and be isolated in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine anti-viral activity using the standard tests described herein, or using other similar tests which are well known in the art. Specific and preferred values listed below for radicals, substituents, and ranges, are for illustration only; they do not exclude other defined values or other values within defined ranges for the radicals and substituents. Specifically, (C₁-C₄)alkyl can be methyl, ethyl, propyl, isopropyl, butyl, iso-butyl, or sec-butyl; (C₃-C₆)cycloalkyl can be cyclopropyl, cyclobutyl, cyclopentyl, or cyclohexyl; hydroxy(C₁-C₄)alkyl can be hydroxymethyl, hydroxyethyl, 3-hydroxypropyl, 2-hydroxypropyl, or 4-hydroxybutyl.

As used herein, "treating" a subject afflicted with a medical condition shall mean slowing, stopping or reversing the condition's progression. In the preferred embodiment, treating a subject afflicted with a disorder means reversing the disorder's progression, ideally to the point of eliminating the disorder itself. As used herein, ameliorating a disorder and treating a disorder are equivalent. The treatment of the present invention may also, or alternatively, relate to a prophylactic administration of said cells. Such a prophylactic administration may relate to the "prevention" of any given medical condition, or the prevention of development of said condition, whereby prevention or prophylaxis is not to be construed narrowly under all conditions as absolute prevention. Prevention or prophylaxis may also relate to a reduction of the risk of a subject developing any given medical condition, preferably in a subject at risk of said condition.

Autophagy is the natural, regulated mechanism of the cell that disassembles unnecessary or dysfunctional components. It allows the orderly degradation and recycling of cellular components. Four forms of autophagy are commonly described: macroautophagy, also termed autophagy, runs in a basal (constitutive) form within the cell. Autophagy can be induced by stressors e.g. nutrient starvation or energy depletion. Both types of autophagy utilize different protein complexes for their maintenance (Murrow & Betnath, 2018). Additional forms of autophagy comprise microautophagy and chaperone-mediated autophagy (CMA).

In the context of the invention, the compounds of the invention inhibit or reduce the level of basal autophagy (or macroautophagy) in a cell whereas induced autophagy is practically not inhibited or to a lesser extent, such as at negligible levels, meaning that the influence of the compound on induced autophagy does not have as significant an effect, or does not have any measurable effect on cellular mechanisms and functions associated with induced autophagy.

In this context, the term "induced autophagy" in particular relates to autophagy mechanisms that are used to produce amino acids following starvation, whereas basal autophagy is important for constitutive turnover of cytosolic components.

Macroautophagy is the main pathway, used primarily to eradicate damaged cell organelles or unused proteins. First the phagophore engulfs the material that needs to be degraded, which forms a double membrane known as an autophagosome, around the organelle marked for destruction. The autophagosome then travels through the cytoplasm of the cell to a lysosome, and the two organelles fuse (so-called autophagolysosome or 'autolysosome'). Within the autolysosome, the contents of the autophagosome are degraded via acidic lysosomal hydrolases.

Induced autophagy, on the other hand, uses different mechanisms for degradation of cellular components and includes microautophagy and chaperone-mediated autophagy (CMA). Microautophagy involves the direct engulfment of cytoplasmic material into the lysosome. This occurs by invagination, meaning the inward folding of the lysosomal membrane, or cellular protrusion. The microautophagic pathway is especially important for survival of cells under conditions of starvation, nitrogen deprivation, or after treatment with rapamycin.

Chaperone-mediated autophagy, or CMA, is a very complex and specific pathway, which involves the recognition by the hsc70-containing complex. This means that a protein must contain the recognition site for this hsc70 complex which will allow it to bind to this chaperone, forming the CMA- substrate/chaperone complex. This complex then moves to the lysosomal membrane-bound protein that will recognise and bind with the CMA receptor, allowing it to enter the cell. Upon recognition, the substrate protein gets unfolded and it is translocated across the lysosome membrane with the assistance of the lysosomal hsc70 chaperone. CMA is significantly different from other types of autophagy because it translocates protein material in a one by one manner, and it is extremely selective about what material crosses the lysosomal barrier. CMA is increased upon genotoxic stress and starvation/nutrient depreciation.

The invention relates to anti-herpesvirus guanosine analogues for use as a medicament to inhibit basal autophagy in the treatment and/or prevention of a medical condition in which basal autophagy is involved in the pathogenesis of said condition. In this context, a medical condition in which basal autophagy is involved in the pathogenesis of said condition is a condition that is caused or promoted by ongoing or enhance basal autophagy in cells that are involved in the pathogenesis of the respective condition. In other words, in such medical conditions the pathogenesis and/or disease progression is promoted by ongoing or enhanced autophagy, whereas an inhibition of basal autophagy slows down or stops disease progression and can even lead to or contribute to eradication of the condition.

The person skilled in the art is able to identify such conditions either based on known disease mechanisms or by applying established test methods for identifying such diseases.

For example, it is known that basal autophagy contributes to disease progression in certain infectious diseases. As used herein "infectious disease" comprises all diseases or disorders that are associated with bacterial and/or viral and/or fungal infections. In several infectious diseases known to or identifiable by the person skilled in the art basal autophagy occurs in cells that are infected with the infectious agent, such as a specific bacterium, virus or fungus, and supports and/or propagates infection, for example by promoting replication of the infectious agent. This is in particular the case in infections with Herpes Simplex Virus 2 (HSV-2) and Epstein-Barr-Virus (EBV), Influenza A virus, Hepaptitis C virus (HCV), *Staphylococcus aureus* (*S*. *aureus*),) and Coronaviruses (CoV), including severe acute respiratory syndrome-related coronavirus (SARS-CoV), Middle East respiratory syndrome-related coronavirus (MERS-CoV), human coronavirus OC43 (hCoV-OC43) and hCoV-229E rely on autophagy and participation of the basal form is suspected. In theses infectious diseases, propagation of the infection correlates with the replication of the infectious agent in the infected host cell, which is promotes or supported by ongoing (basal) autophagy.

Similarly, in certain types of cancer it has been observed that basal autophagy is increased to promote cancer disease progression by promoting several mechanisms involved in cancer disease progression and development, such as for example the transformation of cells to cancer cells, cancer cell proliferation, cancer cell survival, cancer cell migration, motility and formation of metastasis.

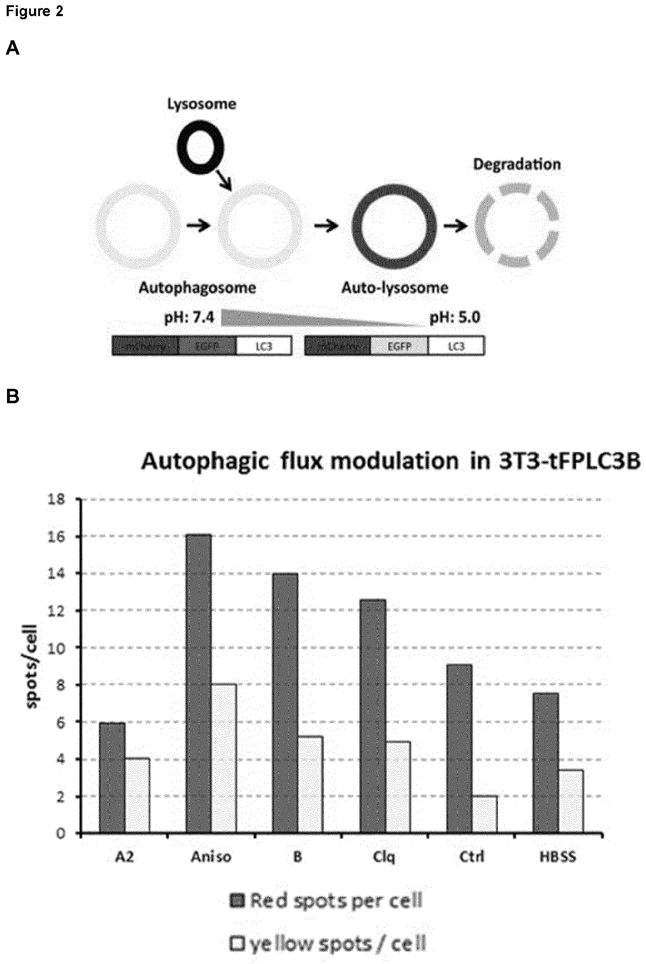

In general, it is known to the skilled person how to identify medical conditions in which basal autophagy is involve in the pathogenesis of said condition, for example by employing the RFP-GFP-LC3B tandem reporter construct described in the context of the experiment displayed in Figure 2. For example, when isolating cells from a subject suspected of suffering from a medical condition according to the present invention, such as a medical condition in which basal autophagy is involved in the pathogenesis of said condition, and corresponding cells from a healthy control subject, these cells can be analyzed for their rate of basal autophagy by employing the RFP-GFP-LC3B tandem reporter construct and measuring the rate of autophagosome and autophagolysosome formation as a measure for basal autophagy. An increased rate of autophagolysosome formation in the diseased cells as compared to healthy control cells identifies a medical condition in which basal autophagy is involved in the pathogenesis of said condition. Other established methods for identifying such conditions by established experimental protocols are known to the person skilled in the art.

Further assays for determination of the degree and the phase of autophagy comprise the CytoID2 test (Enzo Lifesciences), the LC3-I/II conversion Western Blot and the WIPI-2 GFP assay of early autophagy events especially in a high content analysis mode. In the CytoID2 assay a fluorescent dye specifically associates with autophagosomes. Thus, the degree of membrane bound fluorescence correlates with the number of autophagosomes.

LC3 is the most widely used autophagosome marker because the amount of LC3-II reflect the number of autophagosomes and autophagy-related structures. The number of autophagosomes increases by nutrient starvation (deprivation of serum and/or amino acids from cell culture medium), and accordingly the amount of LC3-II also increases. It should be noted, however, that the LC3-II amount at a given time point does not necessarily estimate the autophagic activity, because not only autophagy activation but also inhibition of autophagosome degradation greatly increases the amount of LC3-II. Also, LC3-II can ectopically localize on non-autophagosome structures that are not turned over in the lysosome. To measure the autophagic flux it is essential to determine how much LC3-II (as a model substrate of autophagy) is degraded in a lysosome-dependent manner during a certain time period. Degradation of p62 is another widely used marker to monitor autophagic activity because p62 directly binds to LC3 and is selectively degraded by autophagy. In order to determine the lysosome-dependent degradation, bafilomycin A1 (BafA1), a potent V-ATPase inhibitor, or lysosomal enzyme inhibitors such as E64d and pepstatin A are commonly used. The difference in the amount of LC3-II between samples with and without lysosome inhibitors represents the level of autophagic flux. The Antibodies tend to have greater affinity for LC3-II, thus, the signal ratio of LC3-I and LC3-II does not reflect the amount ratio of cytosolic and membrane-bound LC3. Furthermore, LC3-I can appear very faint depending on the antibodies and cell types. Therefore, the LC3-I amount is not completely reliable as a denominator for quantification of LC3-II (LC3-II/LC3-I). Instead, it is highly recommended to compare the amount of LC3-II with one of the house-keeping proteins, such as tubulin or beta-actin. Further details of monitoring an measuring autophagy have been disclosed by Yoshii et al (Int. J. Mol. Sci. 2017, 18, 1865; doi:10.3390/ijms18091865).

In the LC3 Western Blot, the two different forms of LC3B appearing during the autophagy process are quantified, LC3-I and LC3-II (Mizushima & Kitamura, 2007). LC3-I is a precursor of LC3-II. During elongation and phagophore closure LC3- I is processed and lipidated with PE (phosphatidylethanol amine). The lipidated form associates with the autophagosome membrane. Thus, comparison of the amount of the two forms in comparison to control conditions gives information about the autophagy phase and degree of autophagosome generation. The WIPI-1 protein binds phospholipid PI(3)P and stands at the onset of autophagy. Using a GFP-labelled WIPI-1 the distribution of the label (diffuse cytosolic versus localization at vesicles) reflects the phase and degree of early autophagy (Grotemeier, 2010, Proikas-Cezanne 2011).

The modulation of basal and induced autophagy can be differentiated by the mode of action of the inhibitor underfed and starved conditions. Under fed conditions both inhibitor types can reduce certain early steps in phagosome formation (initiation, phagophore formation or phagophore closure and LC3 association). Under induced conditions basal inhibitors do not influence initial steps leading to induction (MTORC, AMPK complexes) and thus do not alter the autophagosome generation under induced conditions, while inhibitors of the induction pathway do (for these different types of early inhibition compare A1 and A2 in Example 1).

In embodiments of the invention, the medical condition is a cancer that exhibits basal autophagy in support of cancer initiation, cancer cell survival, proliferation, drug resistance and/or motility, which is inhibited by said use of the analogue of the invention. In further embodiments, the medical condition is a cancer that displays elevated levels of basal autophagy compared to a suitable control.

Cancers that fall into these categories can be of any kind, wherein cancer comprises a group of diseases that can affect any part of the body and is caused by abnormal cell growth and proliferation. These proliferating cells have the potential to invade the surrounding tissue and/or to spread to other parts of the body where they form metastasis. Worldwide, there were 14 million new cases of cancer and 8.2 million cancer related deaths in 2012 (World Cancer Report 2014). The majority of cancers is caused by environmental signals involving tobacco use, obesity and infections among others, while around 5-10% are genetic cases. Cancers can be classified into subcategories based on the cell of origin. The most common subcategories are carcinomas from epithelial cells, sarcomas from connective tissue and lymphomas and leukemias from hematopoietic cells. Cancer is associated with a high variety of local and systemic symptoms and cannot be cured in many cases. In light of the high number of new cancer patients and cancer related deaths novel treatment strategies are required.

Cancer according to the present invention refers to all types of cancer or neoplasm or malignant tumors found in mammals, including leukemias, sarcomas, melanomas and carcinomas. Either solid tumors and/or liquid tumors (such as leukemia or lymphoma) may be treated.

Leukemias include, but are not limited to acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcomas include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Melanomas include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Carcinomas include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers include, but are not limited to Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In some embodiments, "tumor" shall include, without limitation, a prostate tumor, a pancreatic tumor, a squamous cell carcinoma, a breast tumor, a melanoma, a basal cell carcinoma, a hepatocellular carcinoma, a choloangiocellular carcinoma, testicular cancer, a neuroblastoma, a glioma or a malignant astrocytic tumor such as glioblastoma multiforme, a colorectal tumor, an endometrial carcinoma, a lung carcinoma, an ovarian tumor, a cervical tumor, an osteosarcoma, a rhabdo/leiomyosarcoma, a synovial sarcoma, an angiosarcoma, an Ewing sarcoma/PNET and a malignant lymphoma. These include primary tumors as well as metastatic tumors (both vascularized and non-vascularized).

In embodiments, the invention relates to a cancer that exhibits an elevated level of basal autophagy compared to a suitable control, wherein a suitable control can be selected by a skilled person depending on the respective type of cancer. For example, the rate of basal autophagy of the respective cancer cells can be compared to the rate of basal autophagy in the untransformed cell type that initially gave rise to the respective cancer cells.

Furthermore, the cancer may be resistant to one or more anticancer drug treatments, such as radiation therapy, chemotherapy or targeted immunotherapies. In this context, resistance to an anticancer drug treatment refers to an unresponsiveness of the respective cancer cells to a particular treatment, which means that growth of the cancer cells and progression of the cancer is not inhibited to a relevant extend by the respective treatment.

The term "resistance" refers to the reduction in effectiveness of a drug or a treatment. The term is used in the context of, for example, pathogens or cancer cells, which have "acquired" resistance to a drug or to another treatment or mechanism that is directed against the pathogen or the cancer cell. Antimicrobial resistance and antineoplastic resistance challenge when an organism or cancer cell is resistant to more than one drug, it is said to be multidrug-resistant. According to the present invention, cancer therapeutic resistance refers to the development of resistance to treatments such as chemotherapy, radiotherapy, irradiation therapy, cell therapy and targeted therapies by cancer cells through different mechanisms. These mechanisms include specific genetic and epigenetic changes in the cancer cell and/or the microenvironment in which the cancer cell resides.

In this context, chemotherapy refers to a category of cancer treatment that uses one or more anti-cancer drugs (chemotherapeutic agents) as part of a standardized chemotherapy regimen. Chemotherapy may be given with a curative intent (which almost always involves combinations of drugs), or it may aim to prolong life or to reduce symptoms (palliative chemotherapy). Chemotherapy is one of the major categories of medical oncology (the medical discipline specifically devoted to pharmacotherapy for cancer). Chemotherapeutic agents are used to treat cancer and are administered in regimens of one or more cycles, combining two or more agents over a period of days to weeks. Such agents are toxic to cells with high proliferative rates - e.g., to the cancer itself, but also to the Gl tract (causing nausea and vomiting), bone marrow (causing various cytopenias) and hair (resulting in baldness).

Chemotherapeutic agents comprise, without limitation, Actinomycin, All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vinblastine, Vincristine, Vindesine, Vinorelbine.

Irradiation or radiation therapy or radiotherapy in the context of the present invention relates to a therapeutic approach using ionizing or ultraviolet-visible (UV/Vis) radiation, generally as part of cancer treatment to control or kill malignant cells such as cancer cells or tumor cells. Radiation therapy may be curative in a number of types of cancer, if they are localized to one area of the body. It may also be used as part of adjuvant therapy, to prevent tumor recurrence after surgery to remove a primary malignant tumor (for example, early stages of breast cancer). Radiation therapy is synergistic with chemotherapy, and can been used before, during, and after chemotherapy in susceptible cancers. Radiation therapy is commonly applied to the cancerous tumor because of its ability to control cell growth. Ionizing radiation works by damaging the DNA of cancerous tissue leading to cellular death. Radiation therapy can be used systemically or locally.

Radiation therapy works by damaging the DNA of cancerous cells. This DNA damage is caused by one of two types of energy, photon or charged particle. This damage is either direct or indirect ionization of the atoms which make up the DNA chain. Indirect ionization happens as a result of the ionization of water, leading to the formation of free radicals, including hydroxyl radicals, which then damage the DNA. In photon therapy, most of the radiation effect is mediated through free radicals. Cells have mechanisms for repairing single-strand DNA damage and double-stranded DNA damage. However, double-stranded DNA breaks are much more difficult to repair, and can lead to dramatic chromosomal abnormalities and genetic deletions. Targeting double-stranded breaks increases the probability that cells will undergo cell death.

The amount of radiation used in photon radiation therapy is measured in gray (Gy), and varies depending on the type and stage of cancer being treated. For curative cases, the typical dose for a solid epithelial tumor ranges from 60 to 80 Gy, while lymphomas are treated with 20 to 40 Gy. Preventive (adjuvant) doses are typically around 45-60 Gy in 1.8-2 Gy fractions (for breast, head, and neck cancers.)

Different types of radiation therapy are known such as external beam radiation therapy, including conventional external beam radiation therapy, stereotactic radiation (radiosurgery), virtual simulation, 3-dimensional conformal radiation therapy, and intensity-modulated radiation therapy, intensity-modulated radiation therapy (IMRT), volumetric modulated arc therapy (VMAT), Particle therapy, auger therapy, brachytherapy, intraoperative radiotherapy, radioisotope therapy and deep inspiration breath-hold.

External beam radiation therapy comprises X-ray, gamma-ray and charged particles and can be applied as a low-dose rate or high dose rate depending on the overall therapeutic approach.

In internal radiation therapy radioactive substance can be bound to one or more monoclonal antibodies. For example, radioactive iodine can be used for thyroid malignancies. Brachytherapy of High dose regime (HDR) or low dose regime (LDR) can be combined with IR in prostate cancer.

A targeted therapy, which can also be referred to as an anti-tumor immunotherapy or cancer immunotherapy attempts to stimulate the immune system to reject and destroy tumors. Immunotherapy encompasses, without limitation, cellular and biopharmaceutical and antibody therapy as well as the use of small molecules inducing a modification of the immune response to tumors.

In embodiments of the invention, the cancer is a tumor or hematological cancer comprising cancer stem cells (CSCs). CSCs are cancer cells that are found within tumors or hematological cancers and possess characteristics associated with normal stem cells, specifically the ability to give rise to all cell types found in a particular cancer sample. CSCs are therefore tumorigenic (tumor-forming), perhaps in contrast to other non-tumorigenic cancer cells. CSCs may generate tumors through the stem cell processes of self-renewal and differentiation into multiple cell types. Such cells are hypothesized to persist in tumors as a distinct population and cause relapse and metastasis by giving rise to new tumors. Therefore, development of specific therapies targeted at CSCs holds hope for improvement of survival and quality of life of cancer patients, especially for patients with metastatic disease. Existing cancer treatments have mostly been developed based on animal models, where therapies able to promote tumor shrinkage were deemed effective. However, animals do not provide a complete model of human disease. In particular, in mice, whose life spans do not exceed two years, tumor relapse is difficult to study. The efficacy of cancer treatments is, in the initial stages of testing, often measured by the ablation fraction of tumor mass (fractional kill). As CSCs form a small proportion of the tumor, this may not necessarily select for drugs that act specifically on the stem cells. Therefore, conventional chemotherapies may kill preferentially differentiated or differentiating cancer cells, which form the bulk of the tumor but do not generate new cells. A population of CSCs, which gave rise to it, could remain untouched and cause relapse.

Another aspect of the invention relates to a pharmaceutical composition prepared for administration to a subject and which include a therapeutically effective amount of one or more of the compounds of the invention, namely anti-herpesvirus guanosine analogues. The therapeutically effective amount of a compound of the invention will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of" and "administering a" compound should be understood to mean providing a compound, a prodrug of a compound, or a pharmaceutical composition as described herein. The compound or composition can be administered by another person to the subject (e.g., intravenously) or it can be self-administered by the subject (e.g., tablets).

Any references herein to a compound for use as a medicament in the treatment of a medical condition also relate to a method of treating said medical condition comprising the administration of a compound, or composition comprising said compound, to a subject in need thereof, or to the use of a compound, composition comprising said compound, in the treatment of said medical condition.

Dosage can be varied by the attending clinician to maintain a desired concentration at a target site (for example, the lungs or systemic circulation). Higher or lower concentrations can be selected based on the mode of delivery or route of administration, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The present invention also relates to a method of treatment of subjects suffering from the various medical conditions disclosed herein. The method of treatment comprises preferably the administration of a therapeutically effective amount of a compound disclosed herein to a subject in need thereof.

In the context of the present invention, the term "medicament" refers to a drug, a pharmaceutical drug or a medicinal product used to diagnose, cure, treat, or prevent disease. It refers to any substance or combination of substances presented as having properties for treating or preventing disease. The term comprises any substance or combination of substances, which may be used in or administered either with a view to restoring, correcting or modifying physiological functions by exerting a pharmacological, immunological or metabolic action, or to making a medical diagnosis. The term medicament comprises biological drugs, small molecule drugs or other physical material that affects physiological processes.

Administration of the compounds of the invention can be individual as mono-therapy or in combination with one or more other therapeutic drugs, for example anti-cancer therapies/drugs. In the context of the present invention the terms "in combination" or "combined administration" indicate that an individual that receives the compound according to the present invention also receives other therapies, which does not necessarily happen simultaneously, combined in a single pharmacological composition or via the same route of administration. "In combination" therefore refers to the treatment of an individual suffering from a medical condition as disclosed herein with more than one therapeutic compounds. Combined administration encompasses simultaneous treatment, co-treatment or joint treatment, whereby treatment may occur within minutes of each other, in the same hour, on the same day, in the same week or in the same month as one another.

### FIGURES

The invention is further described by the following figures. These are not intended to limit the scope of the invention but represent preferred embodiments of the invention and are provided for greater illustration.

### Description of the figures:

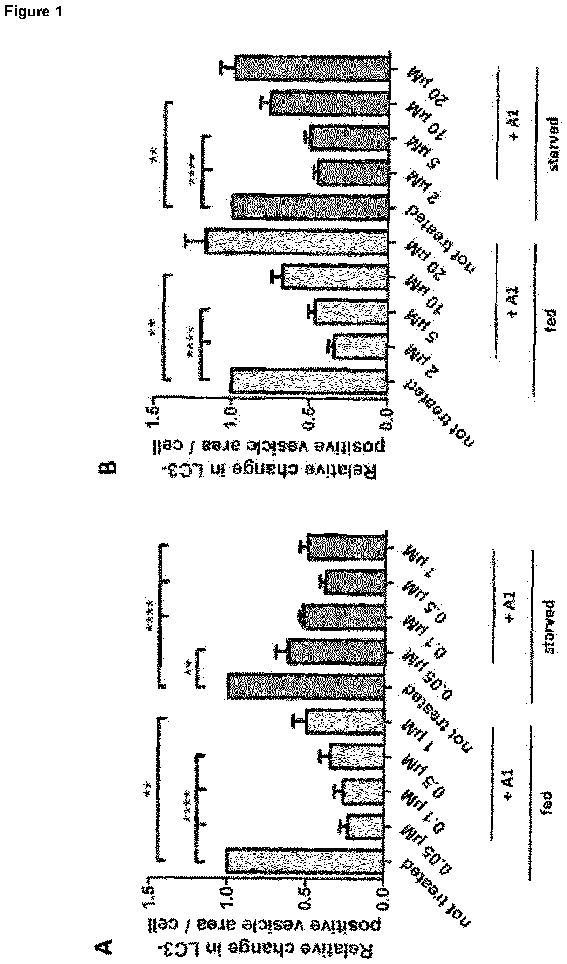

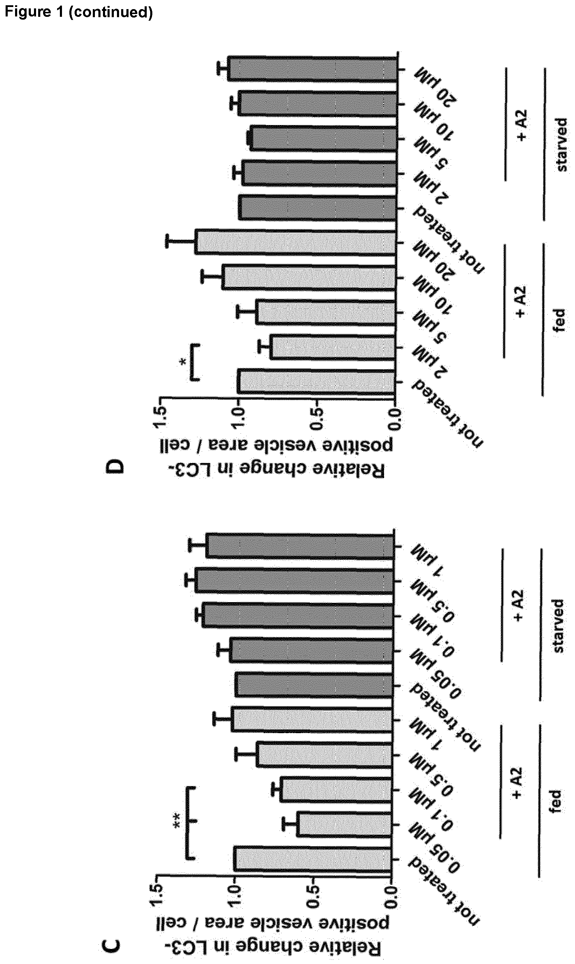

**Figure 1****:** *Dose response in autophagy-modulation of commercial, bioactive compounds.*
   (A, B, C, D) Concentration-dependent effects of compound A1 (compound A1 is Anisomycin; A and B) and compound A2 (compound A2 is aciclovir; C and D) on the LC3-positive vesicle area per cell in fed or starved A549 GFP-LC3B reporter cells. Relative changes are calculated in comparison to the respective control. Fed cells were cultivated for 3 h and starved cells for 1.5 h. Statistical indication according to unpaired two-sided t-test as follows: **** p < 0.0001, *** 0.0001 < p < 0.001, ** 0.001 < p < 0.01, * 0.01 < p < 0.05.
   Compounds A1 and A2 represent early inhibitors of basal autophagy as the amount of membrane-bound GFP-LC3 is significantly decreased to 20% and 60%, respectively, compared to the control. Interestingly, decreasing concentrations of the compounds in fed cells elicit a stronger response compared to standard concentrations applied in initial experiments and screenings (figure 1 B and D compared to figure 8 A and C). The inhibitory effect on autophagy might yet be even stronger with a further reduction of the concentrations. In starved cells, for compound A1 a minimal concentration of 0.5 µM shows the strongest inhibitory effect on autophagy (figure 1 A). Surprisingly, unlike A1 substance A2 does not inhibit induced autophagy (starved cells) and, thus, might exploit a different, unknown mechanism to influence basal autophagy with no interference of induced autophagy (figure 1 C and D).
**Figure 2****:** *Modulation of the autophagic flux in mouse fibroblasts.*
   A. The 3T3 fibroblasts harbor a RFP-GFP-LC3B tandem reporter construct to follow biosynthesis and maturation of autophagic particles, by enabling the discrimination/quantification of autophagosomes/phagophores, autolysosomes and total autophagic particles. GFP, but not RFP, loses its fluorescent properties due to the acidic conditions in autolysosomes (scheme adapted from Hundeshagen et al., 2011). B. Fluorescent images taken from 120-160 cells were analyzed for vesicular, red spot numbers and colocalization of punctate red/green signals. Average values are shown. Image acquisition was performed with the ImageXpress automatic microscope (Molecular Devices); image evaluation was done with the Gen5 software (BioTek). In this assay, red spot numbers correlate with total LC3 autophagic particles, yellow colocalization parallels the number of autophagosomes/phagophores.
   Compared to the control, total LC3 containing autophagic particle numbers are increased upon addition of 2 µM Chloroquine (Clq), B, and Anisomycin (Aniso, A1). Activation is observed in B and Anisomycin treatments, which is expected from earlier results and survey/interpretation of published experiments; late inhibition, which is accompanied by accumulation of autophagosomes, is noted upon addition of Chloroquine (Clq). Interestingly, incubation with compound A2, which is aciclovir, reduces total autophagic particle numbers (by 40%) and the number of autolysosomes (4fold; calculated by subtraction), indicating an early inhibition of autophagy in mouse fibroblasts, too (compare examples 1 and 2). The slight increase in autophagosome/phagophores upon addition of A2 might point to a putative target site or step for A2 action within the autophagy process. Interestingly, Anisomycin (A1) turned out to be a putative inducer in mouse 3T3 fibroblasts at the given concentration but represents an inhibitor in human epithelial A549 (compare figures 1 and 2).

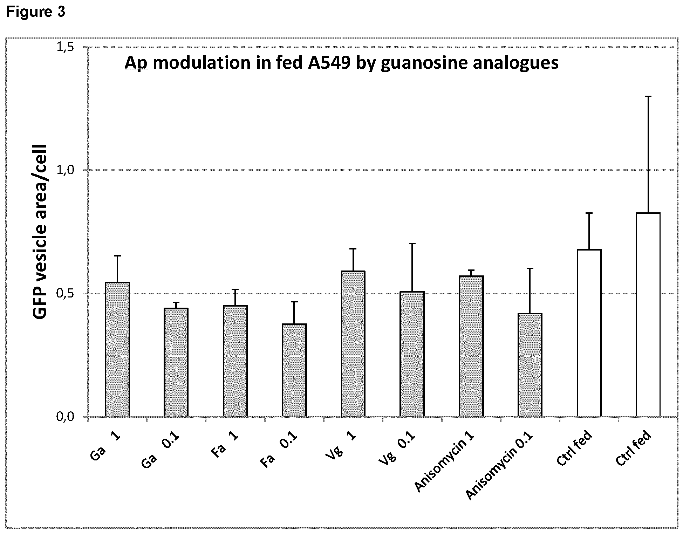

**Figure 3****:** *Modulation of basal autophagy in fed A549 cells.*
   Guanosine analogues diminish autophagy in fed A549 cells to a different extent. Fed A549 cells harboring GFP-LC3B reporter were treated with the indicated concentrations (µM) of ganciclovir (Ga), famciclovir (Fa), valganciclovir (Vg) and anisomycin. 3h later vesicular GFP was determined by automatic fluorescent microscopy. Famciclovir reduces LC3 fluorescence 2-fold compared to the control.

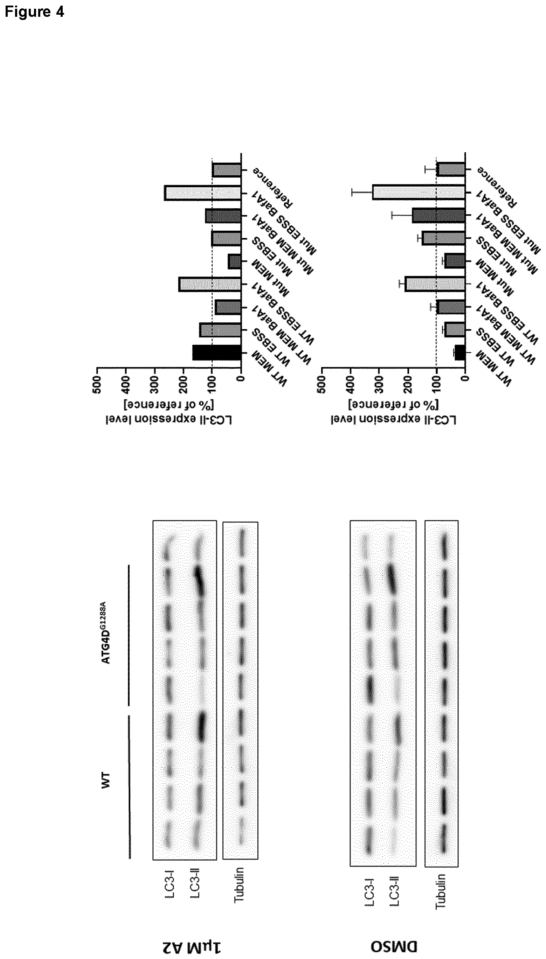

**Figure 4****:** *Modulation of basal autophagy in wt and mutant ATG4D ^{G1288A} Lagotto Romagnolo dog fibroblasts representing a model system for NVSD.*
   Figure 4 shows the effect acyclovir (A2) on basal and induced LC3-II levels in wt and mutant ATG4D^{G1288A} Lagotto Romagnolo dog fibroblasts. Basal and induced autophagy are compared in wt and mutant cells, either after treatment with DMSO or 1 µM A2.

Induction (starvation in Earle's Balanced Salt Solution (EBSS)) increases autophagy activity, as detected by formation of the marker LC3-II. Since autophagy consists of several steps and the last one is a degradation step, a block must be set in order to assess the proportion of the synthesis/maturation step more precisely. To this end, Bafilomycin A1 (BafA1) is added to the cells. BafA1 inhibits the fusion of autophagosomes to autolysosomes and then the degradation of the vesicle content. The comparison +/- Baf A1 is used to measure the autophagy flux. Since BafA1 inhibits the degradation of vesicle contents, LC3-II determination in WBs can be used to determine the rate of autophagosome production/maturation (corresponding to the early to middle part of the autophagy process) and the rate of autolysosomal degradation (late phase), i.e. the total degradation capacity.

In DMSO treated control cells basal autophagy is increased 2-fold in mutant cells compared to wt cells (compare for example DMSO lines 3 and 7, right). A2 diminishes LC3-II levels of basal autophagy in mutant LR cells to level observed in wt cells (compare A2 lines 3,7 densitometric evaluation on the right), but leaving LC3 levels induced by starvation nearly unaltered (compare A2 lanes 4 and 8). Furthermore, less LC3 I seems to be present upon A2 addition in treated mutant cells (Western blot LC3-I lanes 5 DMSO and A2).

A reference sample was loaded onto each gel to compare the results of different gels. The reference sample consists of WT cells grown in full medium conditions without any treatment. Graphs show expression levels of LC3-II normalized to the loading control (tubulin) and the reference sample and represent mean + SEM.

### EXAMPLES

The invention is further described by the following examples. These are not intended to limit the scope of the invention but represent preferred embodiments of the invention and are provided for greater illustration.

As described in the figures, concentration-dependent effects of compound A1 (Anisomycin) and compound A2 (aciclovir) were assessed with respect to the LC3-positive vesicle area per cell in fed or starved A549 GFP-LC3B reporter cells. As shown in Figure 1, compounds A1 and A2 represent early inhibitors of basal autophagy.

Modulation of the autophagic flux was also assessed in mouse fibroblasts. Compared to the control, total LC3 containing autophagic particle numbers are increased upon addition of 2 µM Chloroquine or Anisomycin. Incubation with aciclovir reduces total autophagic particle numbers and the number of autolysosomes, indicating an early inhibition of autophagy in mouse fibroblasts.

The Results of the Examples are described in more detail in the detailed description of the figures.

### Methods employed in the Examples

### High-content analysis using automated fluorescence microscopy

For the single reporter analyses of figure 1 A549 stably transduced with a retroviral GFP LC3 fusion vector were used, for the tandem reporter analyses of figure 2 NIH mouse fibroblasts stably transduced with a retroviral RFP-GFP-LC3B fusion construct were employed. Cells were seeded on 96-well plates at a density of 3.5 x 10⁴ cells per well in 100 µL of their respective growth medium (DMEM for NIH3T3 and F-12K for A549, respectively). The plates were left at room temperature for one hour without moving them to guarantee an even distribution of the cells on the well bottom and then cultured over night at 37 °C and 5% CO₂.

On the next day, compounds were added at the appropriate concentration in either 100 µL of their normal fed medium or 100 µL of Hank's balanced salt solution (HBSS) to induce starvation and then cultured at 37 °C and 5% CO₂ for 3 h or 1.5 h respectively. Subsequently, cells were carefully washed once with phosphate buffered saline (PBS), fixed in 4% formaldehyde for 20 min at room temperature and washed again twice with PBS. Nuclear staining was performed using 0.05% Hoechst 33342 solution in PBS for 10 min at room temperature.

After another two times of washing in PBS, cells could be stored in PBS at 4 °C and were ready for measurement with the automatic fluorescence microscope (ImageXpress Micro XLS Widefield High-Content Imaging system). For each well, images at nine different sites were taken automatically (60x objective, magnification 600x). Exposure times were 150 ms for DAPI, 1000 ms for FITC (single reporter LC3) and 150 ms for DAPI, 1000 ms for FITC, 5000 ms for Texas Red (tandem reporter analysis).

### Quantification of GFP-LC3 puncta for autophagy modulation

Duplicate samples were analyzed comprising 500-1000 cells/well, which represents a coverage of at least 2%/well. Analyses were performed using the Transfluor modul of MetaXpress 6 software (Molecular Devices) with the following settings: vesicle width 0.5 µm - 5 µm and necessary intensity above local background 2000 gray levels determined from the FITC or 500 gray levels determined from the Texas Red image. Size of nuclei 5 µm - 30 µm and necessary intensity above local background 2000 gray levels determined from the DAPI image.

### Quantification of tandem reporter (GFP-RFP-LC3) for autophagy modulation and monitoring of autophagic flux

NIH/3T3 cells stably expressing RFP-GFP-LC3 were treated to induce or inhibit autophagy (for control) and with the compound in question and taken for image acquisition (described above). Between 120-160 cells were analyzed in each quantification for vesicular, red spot numbers and colocalization of punctate red/green signals using the Gen5 software package (BioTek). Average values are shown.

In this assay, red spot numbers correlate with total LC3 autophagic particles, yellow colocalization parallels the number of autophagosomes/phagophores.

### Cell culture and starvation experiments using wt and mutant ATG4D ^{G1288A} Lagotto Romagnolo dog fibroblasts

Fibroblasts from affected dogs and age-, breed-, and sex-matched controls were cultured in high glucose Dulbecco's Modified Eagle Medium (DMEM 41965-039 Gibco) with 10% fetal bovine serum (FBS 10500056 Gibco) and penicillin-streptomycin (PenStrep15140122 Gibco). During second or third passages, cells were seeded at equal density onto Petri dishes and additionally onto glass cover slips. At semi-confluency, the cells were incubated with DMSO or A2 (1µM) for two hours either in fresh medium, with and without 0.4 mM bafilomycin (Bafilomycin A1 BioViotica NSC381866), or in starvation medium (Earl's Balanced Salt Solution (EBSS 24010043 Gibco) with and without 0.4 mM bafilomycin. The cells were harvested and lysed with RIPA buffer containing 0.5 M Tris-HCI, pH 7.4, 1.5 M NaCl, 2.5% deoxy-cholic acid, 10% NP-40, 10 mM EDTA (R0278 Sigma), and an EDTA-free protease inhibitor cocktail (SigmaFAST S8830). The lysates were subjected to Western blotting (WB) with anti-LC3 antibody (anti-LC3 L7543 Sigma-Aldrich). Beta actin was used as the loading control for relative quantification of the blots (anti-beta Actin PA5-16914 Thermo Fischer Scientific). Signals were visualized by enhanced chemiluminescence and further analyzed by densitometry. The blotting experiments were conducted as 2 biological replicates for DMSO treatment and 1 biological replicate for A2 treatment.

## Claims

1. Anti-herpesvirus guanosine analogue for use as a medicament to inhibit basal autophagy in the treatment and/or prevention of a medical condition in which basal autophagy is involved in the pathogenesis of said condition.

2. The anti-herpesvirus guanosine analogue for use as a medicament according to the preceding claim, wherein said analogue is selected from the group consisting of aciclovir, penciclovir, ganciclovir and/or analogues, derivatives, esters or prodrugs thereof, such as valaciclovir, valganciclovir and famciclovir.

3. The anti-herpesvirus guanosine analogue for use as a medicament according to any one of the preceding claims, wherein said analogue inhibits basal autophagy but does not inhibit induced autophagy or inhibits induced autophagy at negligible levels.

4. The anti-herpesvirus guanosine analogue for use as a medicament according to any one of the preceding claims, wherein the medical condition is an infectious disease, in which basal autophagy in the host cell of a disease-causing infectious agent supports infection and/or propagation of the infection, which is inhibited by said use of the analogue.

5. The anti-herpesvirus guanosine analogue for use as a medicament according to the preceding claim, wherein the infectious disease is selected from the group consisting of infections with Hepaptitis C virus (HCV), Herpes Simplex Virus 2 (HSV-2), Epstein-Barr-Virus (EBV) and Coronaviruses (CoV), including severe acute respiratory syndrome-related coronavirus (SARS-CoV), Middle East respiratory syndrome-related coronavirus (MERS-CoV), human coronavirus OC43 (hCoV-OC43) and hCoV-229E.

6. The anti-herpesvirus guanosine analogue for use as a medicament according to claim 4, wherein the infectious disease is an infection with SARS-CoV-2.

7. The anti-herpesvirus guanosine analogue for use as a medicament according to any one of claims 1 to 3, wherein the medical condition is cancer, and wherein
a. the cancer exhibits basal autophagy in support of cancer initiation, cancer cell survival, proliferation, drug resistance and/or motility, which is inhibited by said use of the analogue, and/or
b. the cancer has elevated levels of basal autophagy compared to a suitable control.

8. The anti-herpesvirus guanosine analogue for use as a medicament according to the preceding claim, wherein:
- the cancer cells are resistant to one or more anticancer treatments, such as radiation therapy, chemotherapy or targeted immunotherapies; and/or
- the cancer cells exhibit elevated levels of autophagy as a cell survival-enhancing protective response to tumor microenvironmental stress, such as endoplasmic reticulum (ER) stress, hypoxia, oxidative stress and/or glucose deprivation;
- wherein said cancer is preferably selected from breast cancer, colorectal cancer, esophageal cancer, glioblastoma, hepatocellular carcinoma, leukemia and MCL, lung cancer, Pancreatic adenocarcinoma, prostate cancer, renal cell carcinoma or ovarian cancer.

9. The anti-herpesvirus guanosine analogue for use as a medicament according to claim 7, wherein the cancer is a tumor or hematological cancer comprising cancer stem cells (CSCs), wherein the CSCs:
- exhibit basal autophagy in support of CSC pluripotency, CSC proliferation and/or CSC migration and/or invasion,
- exhibit elevated levels of basal autophagy compared to a suitable control, and/or
- are resistant to one or more anticancer treatments, such as radiation therapy, chemotherapy or targeted immunotherapies.

10. The anti-herpesvirus guanosine analogue for use as a medicament according to any one of claim 7 to 9, wherein the use comprises combined administration of said analogue with one or more anticancer treatments, such as radiation therapy, chemotherapy or targeted immunotherapies.

11. The anti-herpesvirus guanosine analogue for use as a medicament according to any one of claims 1 to 3, wherein the condition is a neurodegenerative disease, in which basal autophagy supports pathogenesis of said condition, preferably wherein the condition is Neurodegenerative Vacuolar Storage Disease (NVSD).

12. The anti-herpesvirus guanosine analogue for use as a medicament according to any one of the preceding claims, wherein the subject is a mammal, preferably a human or canine.

13. The anti-herpesvirus guanosine analogue for use as a medicament according to claim 1, wherein the amount of analogue administered to a subject is:
a. below a therapeutically effective dose for treating Herpes Simplex Virus 1 (HSV-1) or varicella-zoster virus (VZV) infection in a subject; and/or
b. insufficient for inhibiting viral thymidine kinase in a subject infected with Herpes Simplex Virus 1 (HSV-1) or varicella-zoster virus (VZV).

14. The anti-herpesvirus guanosine analogue for use as a medicament according to any one of the preceding claims, wherein the amount of analogue administered to a subject is at least 5 times lower, preferably 10 times, more preferably 20 times lower, than (a) a therapeutically effective dose for treating HSV-1 or VZV infection in a subject, or (b) a dose effective in inhibiting viral thymidine kinase in a subject infected with HSV-1 or VZV.

15. The anti-herpesvirus guanosine analogue for use as a medicament according to the preceding claims, wherein:
a. the dose of analogue, preferably aciclovir, administered is less than 5 mg/kg subject body weight per dose, for example about 3 mg/kg subject body weight or less per dose, preferably 1 mg/kg subject body weight or less per dose, more preferably 0.5 mg/kg subject body weight or less per dose; and/or
b. wherein the analogue, preferably aciclovir, is administered in a liquid pharmaceutical composition at a concentration of less than 25 mg/ml, such as 10 mg/ml or less, preferably 2.5 mg/ml or less, more preferably 1 mg/ml or less, or is administered in a solid pharmaceutical composition comprising less than 200 mg, such as 100 mg or less, preferably 50 mg or less, more preferably 10 mg or less.
